**Europäisches Patentamt**

**European Patent Office**

**Office européen des brevets**

(19)

(11) **EP 1 102 065 A2**

(12) **EUROPÄISCHE PATENTANMELDUNG**

(43) Veröffentlichungstag:
**23.05.2001 Patentblatt 2001/21**

(51) Int Cl.7: **G01N 33/24**

(21) Anmeldenummer: **00124708.9**

(22) Anmeldetag: **11.11.2000**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **15.11.1999 DE 19954783**
**10.11.2000 DE 10055821**

(71) Anmelder:
• **Flick, Gerhard, Prof. Dr.**
**17033 Neubrandenburg (DE)**

• **Harig, Peter Michael, Dipl. Chem.**
**17033 Neubrandenburg (DE)**

(72) Erfinder:
• **Flick, Gerhard, Prof. Dr.**
**17033 Neubrandenburg (DE)**
• **Harig, Peter Michael, Dipl. Chem.**
**17033 Neubrandenburg (DE)**

(74) Vertreter: **Voss, Karl-Heinz**
**Kruseshofer Strasse 20**
**17036 Neubrandenburg (DE)**

(54) **Verfahren zur Ermittlung der Verfügbarkeit von Stickstoff und/oder Kohlenstoff in Böden und von Erden, Sedimenten und Komposten für das Pflanzenwachstum zur Quantifizierung der Stickstoffdüngung**

(57) Verfahren zur Ermittlung der Verfügbarkeit von Stickstoff und/odcr Kohlenstoff in Böden und von Erden, Sedimenten und Komposten für das Pflanzenwachstum zur Quantifizierung der Stickstoffdüngung Die Erfindung betrifft ein Verfahren zur Ermittlung der aktuellen, Bioverfügbarkeit von Stickstoff und/oder Kohlenstoff in landwirtschaftlich genutzten Böden, von Erden, Sedimenten, Komposten usw. hinsichtlich der gezielten Pflanzenernährung und der Minderung der Umweltbelastung durch diese Nährstoffe.

Aufgabe der Erfindung ist es, ein kostengünstiges und effizientes Verfahren zu gestalten, dass die Bestimmung des Stickstoff- und/oder Kohlenstoffbedarfs von Böden, Erden, Komposten und dergleichen hinsichtlich eines optimalen Pflanzenwachstums, geringster Umweltbelastung und damit eine optimale Stickstoffdüngung ermöglicht.

Erfindungsgemäß wird das Nachlieferungspotential für pflanzenverfügbaren bzw. organisch gebundenen N und/oder C durch Messung der Gehalte in der wasserlöslichen organischen Substanz (WOS) und wasserunlöslichen organischen Substanz(WULOS) ermittelt, die Bevorratungseigenschaften durch die Humusausstattung mittels Messung des organischen Kohlenstoffgehaltes $C_{gesamt}$ erfasst und die Umsatzeigenschaften des Bodens durch Messung der Bodentextur und der Wasserkapazität in Kombination mit den Klimafaktoren ermittelt Die analysierten Proben werden zusätzlich mittels Reflexionsspektroskopie und/oder Transmissionsspektroskopie im Wellenlängenbereich von 400 nm bis 2500 nm vermessen, die Messgrößen in Form der Absorptionswerte bei bestimmten Wellenlängen mittels Regressionsgleichungen kalibriert und quantitativ nach dem Gehalt an Stickstoff und Kohlenstoff durch den rechnerischen Vergleich der Werte aus der/den nasschemisch analysierten Fraktion(en) ausgewertet und die ermittelten Beziehungsgrößen in Form von Gruppen- oder Standardkalibrationen für die quantitative Auswertung weiterer Probenmesswerte aus der Reflexionsspektroskopie oder Transmissionsspektroskopie im Wellenlängenbereich von 400 nm bis 2500 nm genutzt und abschließend durch Vergleich mit den Erträgen eine standortbezogene Einstufung mittels einer Entzugsrechnung und Bilanzierung der Nährstoffe und Kohlenstoff durchgeführt.

Fig. 1

C-N-BIOVERFÜGBARKEIT IM RBU-REGIONALMODELL◦ GLEICHE UMSATZDAUER (WMZ) – UNTERSCHIEDLICHE BEVORRATUNGS-MERKMALE (BODENSCHWERE) UND WOS-C-N IN ABHÄNGIGKEIT VON DER WIRTSCHAFTSBEDINGTEN HUMUSREPRODUKTION

EP 1 102 065 A2

## Beschreibung

Die Erfindung betrifft ein Verfahren zur Ermittlung der aktuellen, Bioverfügbarkeit von Stickstoff und/oder Kohlenstoff in landwirtschaftlich genutzten Böden, von Erden, Sedimenten, Komposten usw. hinsichtlich der gezielten Pflanzenernährung und der Minderung der Umweltbelastung durch diese Nährstoffe.

## [Stand der Technik]

Der Anteil des nutzbaren organischen Stickstoffes des Bodens, von Erden und dergleichen zur Deckung des Nährstoffbedarfs der Kulturpflanzen am gesamten Düngerbedarf kann gegenwärtig noch nicht hinlänglich genau ermittelt werden, so dass trotz weiterentwickelter Untersuchungs- und Anwendungsverfahren zur Ermittlung der Freisetzung aus den Vorräten die Stickstoffdüngung letztendlich nach wie vor einen mehr oder minder zutreffenden Schätzungsanteil beinhaltet.

Es ist bekannt, dass die Mineralisation von Stickstoff und Kohlenstoff im Boden sowie in Erden und dergleichen einerseits vom verfügbaren Nährstoffvorrat ($N_{org}$) im Humus bzw. in der organischen Substanz und andererseits von der Temperatur (T) und der Feuchte (F) in Abhängigkeit von der Witterung, vom Humus (H) als Speichermatrix für schwerer verfügbare Nährstoffvorräte und für Wasser sowie als stoffwechselsteuerndes Strukturelement, von der Korngrößenverteilung (K) und Durchlüftung und schließlich von der Umsetzungsleistung der Bodenmikroflora abhängig ist. Für die Bildung von Vorräten, die Erhaltung eines Gleichgewichts oder dessen Einstellung auf ein niedriges oder höheres Niveau des umsetzbaren organischen Stickstoffs, des $N_{org}$, und parallel dazu des organischen Kohlenstoffs., des $C_{org}$, in Böden und von Erden und dergl. gilt daher folgende Beziehung:

$$N_{org} = N_{org}(T,F,K,H)$$

$$C_{org} = C_{org}\ (T,F,K,H)$$

In den Faktor Humus wird dabei in Kulturböden die bewirtschaftungs- und düngungsbedingte Reproduktion der organischen Bodensubstanz einbezogen. Der Pflanzenbestand trägt in Abhängigkeit von seinem Bedarf zum Entzug des Stickstoffs und zur Rückführung von pflanzenverfügbaren organischem Stickstoff bei.

Alle genannten Größen variieren in der Bodenkrume innerhalb von Klimazonen und von Bodeneinheit zu Bodeneinheit in einem weiten Bereich, so dass von einem landwirtschaftlich genutzten Standort zum anderen mit unterschiedlichen Nachlieferungsmengen von zumindest 20 bis 300 kg/ha und mehr ausgegangen werden kann. An ein und demselben Standort sind jedoch unter gemäßigten Klimabedingungen im Rahmen der üblichen Bodenbewirtschaftung, der Bevorratung bzw. der Abreicherung des $N\text{-}_{org}$ und damit der beeinflussbaren Stickstoffnachlieferung trotz der jahreszeitlichen Schwankungen engere Grenzen gesetzt. Für Substrate, Komposte oder Erden mit unterschiedlichen Bevorratungseigenschaften und Gehaltswerten an Stickstoff und Kohlenstoff gelten für deren Umsetzung Klima- und Standortfaktoren in vergleichbarer Weise, sobald sie dem System Boden angehören.

Dem Nitrat ($NO_3$) kommt als eine der wichtigsten löslichen Stickstoffformen im Boden eine Schlüsselrolle zu. Mit der $N_{min}$ Methode liegt einerseits eine Bestimmungsmethode primär für Nitrat vor, die zur Abschätzung der Nitratverfügbarkeit wertvolle Hinweise liefert, andererseits zeigen sich immer wieder ihre Grenzen, z. B. bei trockenem Klima und flachgründigen Böden.

Obwohl der Stickstoffbedarf und die Mineralisation aus dem organischen Gesamtvorrat während einer Vegetationsphase u.a. wegen der Klimaschwankungen nicht immer kongruent verlaufen, haben Forschungen gezeigt, dass die Kenntnis der verfügbaren Nährstoffe bzw. der entscheidenden Vorratspotentiale und der dort gebundenen Vorräte zu einer gezielten Stickstoffdüngung beitragen kann. Nach dem Stand der Technik kann zu deren Ermittlung von zwei unterschiedlichen Ansätzen ausgegangen werden:

■   Von biologischen Verfahren, bei denen aerobe und/oder anaerobe Umsatzprozesse imitiert werden, wie z. B. invitro Bebrütungsmethoden mit der analytischen Bestimmung der mineralisierten Endprodukte Ammonium bzw. Nitrat (Simulationsmodell),

■   Von der klassischen Bodenanalytik zur Erfassung von unterschiedlich gebundenen Vorräten wie Kalium, Calcium, Phosphor u.a. anorganische Nährstoffe oder von aufzuschließenden Anteilen wie Gesamtstickstoff, Gesamtkohlenstoff u.a. (Kapazitätsmodell)

Für beide Methodengruppen gilt, dass

> Fragen nach den Bindungsstellen und Bindungsmechanismen, d.h. über die Bevorratungseigenschaften des Bodens im weitesten Sinn, die über die Verfügbarkeit der bestimmten Nährstoffe am jeweiligen Standort entscheiden, nicht dezidiert in die Interpretation der Werte eingehen

> gleichlautende Messergebnisse von bestimmten löslichen und unlöslichen organischen Stickstoffvorräten eine unterschiedliche Bedeutung haben können, da die Mobilisierung am jeweiligen Standort verschieden hoch sein kann.

Für die Bodenanalytik ist es seit langem bekannt, dass Pflanzennährstoffe in Böden und dergleichen nach deren Extraktion mit bestimmten wässrigen Auszugslösungen mit Hilfe von chemischen Reagenzien bestimmt werden können. Bei Elementaranalysen der organischen Substanz nach Gesamtstickstoff und Gesamtkohlenstoff werden nach einem Säureaufschluss der Probe titrimetrische oder spektroskopische Standardmethoden angewandt, jedoch finden dazu alternativ auch Verbrennungsverfahren Anwendung. In der Regel werden für eine Reihe von Elementen solche definierten Auszuglösungen verwendet, die auf der Basis von Konventionen in der Beziehung zwischen Gehaltswerten und Ernteergebnissen die bestmögliche Übereinstimmung zeigen. Die auf das Bodengewicht bezogenen Analysenergebnisse werden in Gehaltsklassen eingeteilt, die für einen Großteil aller Böden gelten.

Demgegenüber stehen Verfahren, bei denen für die Bestimmung ein und desselben Elementes mehrere unterschiedlich hergestellte Fraktionen verwendet werden. Diese Verfahren nehmen für sich in Anspruch, dass durch bestimmte Verhältnisse entsprechender Gehaltswerte zueinander Hinweise zum dynamischen Verhalten der Nährstoffe erhalten werden können, wobei auch die Unterschiedlichkeit der Standorte Berücksichtigung findet.

So sind in der DE OS 195 13 310 beispielsweise einige Verfahren beschrieben, die es gestatten, das dynamische Verhalten einzelner Stoffe in Böden, Kompost und dergleichen aufzuzeigen, die hier parallel angewendet werden. Je nach eingesetzter Auszuglösung werden unterschiedliche Anteilsmengen der Nährstoffe extrahiert bzw. aufgeschlossen und damit einer Messung zugänglich.

Die bekannten Laborverfahren zur Bestimmung organischer Bodenkomponenten besitzen aber den Nachteil, das sie einerseits sehr aufwendig sind und andererseits die Komplexität der Beziehungen zur Pflanzenernährung nicht genügend erfassen.

In diesem Sinne ist das Problem einer gezielten Pflanzenernährung mit dem mengenbestimmenden Nährelement Stickstoff und der Versorgung der Böden mit Kohlenstoff bzw. Humus in der landwirtschaftlichen Praxis auch heute noch nicht in befriedigender Weise gelöst, obwohl dies aus Gründen eines effizienten Nährstoffeinsatzes und des Umweltschutzes wünschenswert wäre.

Es hat sich gezeigt, dass neben den unterschiedlichen Bindungsformen und -mechanismen für die Nachlieferung aus dem Stickstoffgesamtvorrat und wasserlöslichem organischen Vorrat ($N_{org}$) in der Natur und damit auch in direkter Konsequenz bei einer Analyse im Labor auch die molekularen Bindungsstellen innerhalb der Bodenaggregate von entscheidender Bedeutung sind, da die Freisetzung des mineralischen Stickstoffs ($N_{min}$) als Pflanzennährstoff über die Stoffwechselleistung syntropher Gesellschaften der Bodenmikroflora in sogenannten Mikrohabitaten erfolgt. Daneben fungiert die überwiegend in Ruhestellung befindliche mikrobielle Biomasse des Bodens selbst als systemimmanenter biologischer Speicher für die gefragten Nährstoffe Stickstoff und Kohlenstoff. In Kenntnis dieser systemaren Zusammenhänge des organischen Bodenstoffwechsels wird angestrebt, so wie durch eine Analytik unterschiedlich gebundener bzw. in unterschiedlichen Auszugslösungen erscheinende Kationen und Anionen des Bodens und deren Verhältnisse zueinander auch verschiedene Fraktionen des $C_{org}$ und des $N_{org}$ unter definierten Bedingungen zu erfassen und die jeweiligen C/N-Verhältnisse) zu bestimmen.

Zur Ermittlung von qualitativen und quantitativen stofflichen Bestandteilen oder chemischen Elementen und Verbindungen sind noch verschiedene andere physikalische Verfahren bekannt, unter anderem die Infarotspektroskopie. Neben der direkten Auswertung des ermittelten Spektrums sind auch indirekte Methoden bekannt geworden. In der DE PS 196 01 950 wird zur Ermittlung der Kunststoffsorten vorgeschlagen, diese mit infrarotem Licht zu bestrahlen und das gemessene Spektrum mit bekannten Spektren von Kunststoffen zu vergleichen. Gemäß der DE OS 197 26 023 werden zur Untersuchung von mineralischen Baustoffen von Modellsubstanzen IR-Spektren aufgenommen, um die Peakerwartungsbereiche zu ermitteln. Die IR-Spektren der unbekannten Baustoffe werden in diesen Erwartungsbereichen aufbereitet und mit den Werten der Modellsubstanzen verglichen.

Die Nutzung der Infarotspektroskopie für die Ermittlung des Gehaltes von bestimmten Elementen wie Stickstoff und Kohlenstoff in Pflanzen ist ebenfalls bekannt, eine Anwendung für die Ermittlung der Nährstoffgehalte in Böden bisher aber nicht.

**[Aufgabe der Erfindung]**

Aufgabe der Erfindung ist es deshalb, ein kostengünstiges und effizientes Verfahren zu gestalten, dass die Bestimmung des Stickstoff- und/oder Kohlenstoffbedarfs von Böden, Erden, Komposten und dergleichen hinsichtlich eines optimalen Pflanzenwachstums, geringster Umweltbelastung und damit eine optimale Stickstoffdüngung ermöglicht.

Erfindungsgemäß wird die Aufgabe gemäß den Merkmalen des Anspruches 1 gelöst.

Untersuchungen haben gezeigt, dass neben den unterschiedlichen Bindungsformen und -mechanismen für die Nachlieferung aus dem Stickstoffgesamtvorrat und wasserlöslichem organischen Vorrat (Norg) in der Natur und damit auch in direkter Konsequenz bei einer Analyse im Labor auch die molekularen Bindungsstellen innerhalb der Bodenaggregate von entscheidender Bedeutung sind, da die Freisetzung des pflanzennährenden mineralischen Stickstoffs (Nmin) über die Stoffwechselleistung syntropher Gesellschaften der Bodenmikroflora in sogenannten Mikrohabitaten erfolgt. Daneben fungiert die überwiegend in Ruhestellung befindliche mikrobielle Biomasse des Bodens selbst als systemimmanenter biologischer Speicher für die gefragten Nährstoffe Stickstoff und Kohlenstoff. In Kenntnis dieser systemaren Zusammenhänge des organischen Bodenstoffwechsels wurde angestrebt, so wie durch eine Analytik unterschiedlich gebundener bzw. in unterschiedlichen Auszugslösungen erscheinende Kationen und Anionen des Bodens und deren Verhältnisse zueinander auch verschiedene Fraktionen des $C_{org}$ und des $N_{org}$ unter definierten Bedingungen zu erfassen und die jeweiligen C/N-Verhältnisse) zu bestimmen.

Als Vorratspotentiale für verfügbar werdenden Stickstoff und Kohlenstoff werden die wasserlöslichen organische Substanzen (WOS) betrachtet, die in geringen Mengen ungebunden im Boden vorkommen, biologisch in der Biomasse und vornehmlich aber adsorptiv an organischen Bindungsstellen wie Humus und an anorganischen Bindungsstellen wie Schluff und Ton gebunden sind.

Die Gehalte an Nährstoffen Böden in Form von wasserlöslichen $N_{org}$ und $C_{org}$ und die verfügbar werdenden Mengen an Stickstoff und Kohlenstoff sind, wie im Stand der Technik dargestellt, von sehr vielen Faktoren abhängig. Zur Bewertung schlägt die Erfindung drei zu erfassende, bedingt variable komplexe Kenngrößen des Bodens vor:

A) Die standortspezifische Bevorratung an gesamter organischer Bodensubstanz und das Nachlieferungspotential des Bodens,
B) die nachlieferbaren bzw. verfügbar werdende Mengen der Vorräte ($N_{org}$, $C_{org}$) - in Form der wasserlöslichen organischen Substanz sowie,
C) die Umsatzeigenschaften in Abhängigkeit vom Klima und bewirtschaftungsbedingten äußeren Faktoren.

Damit kann die Bioverfügbarkeit von N und/oder C mit akzeptablem Aufwand relativ genau ermittelt werden.

Die verfügbare Menge an Nährstoffen in Böden ist, wie dargestellt, von sehr vielen Faktoren abhängig. Überraschend wurde gefunden, dass mittels Reflexionsspektroskopie und/oder Transmissionsspektroskopie im Wellenlängenbereich von 400 nm bis 2500 nm gemessene Absorptionswerte der Proben diese Zusammenhänge annähernd so wiedergeben, wie die nasschemischen Analysemethoden. Besonders aussagekräftig ist hierbei der Infrarotbereich von 800 bis 2500 nm. Die Messgrößen in Form der Absorptionswerte bei bestimmten Wellenlängen werden gemäß der Erfindung mittels Regressionsgleichungen kalibriert und quantitativ nach dem Gehalt an Stickstoff und Kohlenstoff durch den rechnerischen Vergleich der Werte aus der/den nasschemisch analysierten Fraktion(en) ausgewertet. Damit können die ermittelten Beziehungsgrößen in Form von Gruppen- oder Standardkalibrationen für die quantitative Auswertung weiterer Probenmesswerte genutzt werden. Weiterhin hat sich gezeigt, dass auch die Textur und relevanten Qualitätsmerkmale des Bodens, wie dessen Wasserspeicherkapazität und andere, die verfügbare Nährstoffmenge beeinflussende Faktoren durch die erläuterte spektroskopische Bewertung ebenfalls erfasst werden. Dadurch ist eine wesentliche Vereinfachung der Messwerterfassung möglich.

Entsprechend der Erfindung erfolgt dann durch Zuordnung der Messergebnisse und Vergleich mit den Erträgen im Feldversuch oder in der landwirtschaftlichen Praxis eine standortbezogene Einstufung des $N_{org}$ und des $C_{org}$ mittels Entzugsrechnung und Bilanzierung der Nährstoffe Stickstoff und Kohlenstoff, die eine Gegenrechnung von zugeführten und abgeführten Nährstoffmengen einschließt.

Damit kann erreicht werden, Böden und Flächeneinheiten und Regionen mit vergleichbaren Standorteigenschaften räumlich abgegrenzte Konzentrationsmuster zuzuordnen und neben den bewirtschaftsbedingten Individualwerten auch durchschnittliche Richtwerte zu ermitteln. Es wird ein Einteilungsschema für einzelne Standorte möglich, das das Bevorratungs- und Nachlieferungsvermögen für unterschiedliche Bodentypen innerhalb einer abgegrenzten Bodeneinheit angibt und mit dem für diesen Standort unter bekannten Bewirtschaftungsbedingungen die Nachlieferung von Stickstoff und somit das konkrete Nährstoffangebot ermittelt wird.

Zu den nasschemischen und physikochemischen bzw. spektroskopischen Labormethoden für die Aufbereitung und Messung der Gesamtgehalte von Stickstoff und Kohlenstoff und der den Umsatz bestimmenden Rahmengrößen wie Textur und Wasserkapazität sowie der wasserlöslichen Fraktionen werden in den Ansprüchen 2 bis 4 spezielle Verfahrensschritte vorgeschlagen.

Mit der Erfindung sind die verfügbaren Nährstoffmengen von Stickstoff und Kohlenstoff zunächst in der Form von $N_{org}$-Fraktionen und $C_{org}$-Fraktionen und über ein nachfolgendes Ermittlungsverfahren, in welchem sämtliche Messwerte und deren Verhältnisse zueinander berücksichtigt werden, wesentlich einfacher und genauer zu bestimmen. Die Angaben erfolgen in Gewichtseinheiten pro Flächeneinheit als Versorgung oder Fehlbedarf.

**[Beispiele]**

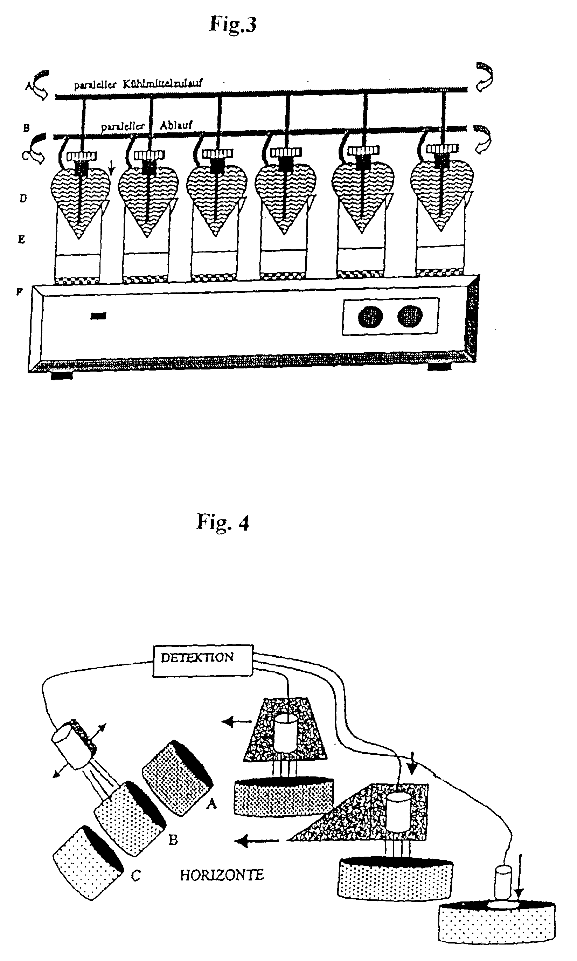

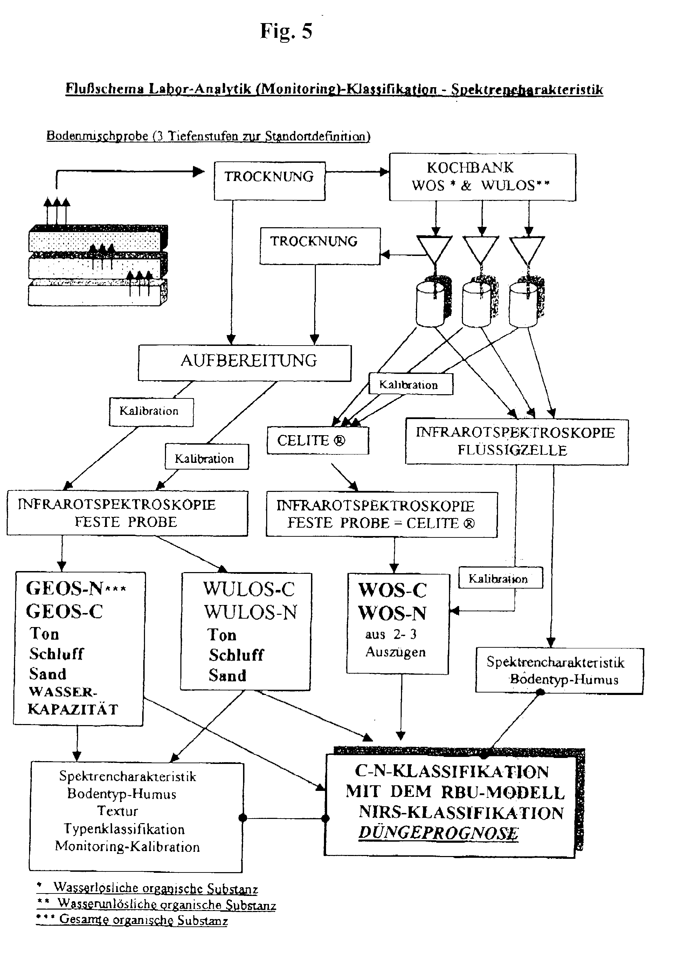

Nachfolgend soll das Verfahren an einem Beispiel erläutert werden. Die Fig. 1 zeigt ein graphisches Modell der zu bestimmenden Größen und deren Beziehungen zueinander. Die Fig.2 zeigt ein Schema der Auswertung und Interpretation aufgrund der entsprechenden Standortdaten, die Figur 3 zeigt eine Kochbank, die Figur 4 die unmittelbare spektroskopische Bodenmessung und Fig. 5 ein Flussschema der Analytik.

Zur Ermittlung der konkreten Werte wird wie folgt verfahren:

1. Bestimmung von wasserlöslichen organischen Substanzen (WOS) im Labor.

Im ersten Schritt wird aus einer bei Temperaturen von bis zu 50 °C luftgetrockneten und auf 2 mm Korngröße gesiebten Probe ein Heißwasserextrakt hergestellt. Dann wird die gleiche Probe herangezogen, um mindestens einen weiteren Auszug, vorzugsweise jedoch zwei weitere wässerige Extrakte mit anorganischen Salz- oder Pufferlösungen herzustellen, und zwar wie folgt:

Extrakt 1 mit kochendem Wasser unter Inkubation der Probe auf einer speziellen Kochbank während einer Stunde oder in Mikrowellen-Aufschlussgeräten in kürzerer Zeit mit oder auch ohne Druckanwendung als die "Fraktion 1", Extrakt 2 mit kochender anorganischer Puffer- oder Salzlösung, vorzugsweise mit 0,04 molarem Phosphatpuffer als "Fraktion 2", Extrakt 3 mit kalter Salzlösung, vorzugsweise 0,01 bis 0,10 molarem Phosphatpuffer als "Fraktion 3" unter Bewegung in einem Laborschüttler während eines Zeitraums von 1 bis 2 h. Die Proben werden vorzugsweise mit Hilfe einer Kochbank extrahiert und die Extrakte nach nasschemischen oder physikochemischen Standardmethoden nach dem Gehalt an organischem Stickstoff und Kohlenstoff analysiert.

| Extraktion | Substanz | Präparation | Messbereich für N |
|---|---|---|---|
| Fraktion 1 | Heißwasserlösliches Material aktive, ruhende, tote Biomasse nur wenig gebunden | 1 h bei 100 ° C | 0.003 % des Bodens = 3 mg N /100 g = um 100 kg N/ ha |
| Fraktion 2 | Das gleiche Material, aber fester an Humus und Feinteile ( Ton u. Schluff) gebunden | Wie 1 jedoch in neutraler Hydrogensalzlösung oder Salzlösung | 0,01 % des Bodens= 10 mg N /100 g = mehr als 300 kg N/ha |
| Fraktion 3 | Kaltwasserlösliches Material Freie, N-haltige Substanz in der Bodenlösung | 1-2 h in der Kälte schütteln | >0,001 % des Bodens = 1 mg N/ 100g |

2. Bestimmung der wasserunlöslichen organischen Substanzen Stickstoff - $N_{gesamt}$ - und Kohlenstoff $C_{gesamt}$ (WULOS) mittels genormter Standardmethoden

| | |
|---|---|
| Gesamtstickstoffgehalt der Probe bzw. des organischen Anteils ( im Bereich von + - 0,1 % des Bodengewichts = 5000 kg N/ha) | Aufschluss durch starke Säuren in der Hitze oder durch trockene Verbrennung ( 1020 °C) |
| Gesamtkohlenstoff der Probe bzw. des organischen Anteils (im Bereich von mehr als + - 1 % des Bodengewichts = 50.000 kg/ha | Aufschluss wie oben |

3. Die Parameter Textur und Wasserspeicherkapazität werden wie folgt mit genormten Standardmethoden bestimmt:

| | |
|---|---|
| Korngrößenverteilung: Prozentanteile von Sand, Schluff und Ton | Sedimentationsanalyse |
| Wasserkapazität: Aufnahme von Wasser bis zur Fließgrenze des Bodens | Feldkapazität |

4. In einem weiteren Schritt wird die "Fraktion 1" und die weiteren Fraktionen 2 und 3 bzw. die vom unlöslichem Probenrest abgetrennten Filtrate oder auch Zentrifugate mit der wassergelösten organischen Substanz in temperaturstabilisierten Küvetten für Flüssigkeiten mittels Nahinfrarotreflexionsspektroskopie oder auch Nahinfrarottransmissionsspektrometrie im Wellenlängenbereich von 800 nm bis 2500 nm vermessen. Alternativ können die Fraktionen auch mit Hilfe von oberflächenaktiven Trägersubstanzen, wie vorzugsweise CELITE ® (2 ml Extrakt zu 1 g Trägersubstanz) zunächst schonend während 2 h bei 50 °C und dann bei 100 °C zur Trockene gebracht und im Wellenlängenbereich wie bei der Analyse in der Flüssigzelle ausgeführt, jedoch in der Pulverküvette, vermessen werden.

5. Nun werden die Messwerte in Form der Absorptionswerte bei bestimmten Wellenlängen mittels Regressionsgleichungen und den in vorhergehenden Schritten ermittelten Messwerten der Analysen mit genormten Standardverfahren kalibriert und die Spektren sämtlicher Proben quantitativ nach dem Gehalt an Stickstoff und Kohlenstoff und den anderen relevanten Parametern ausgewertet.

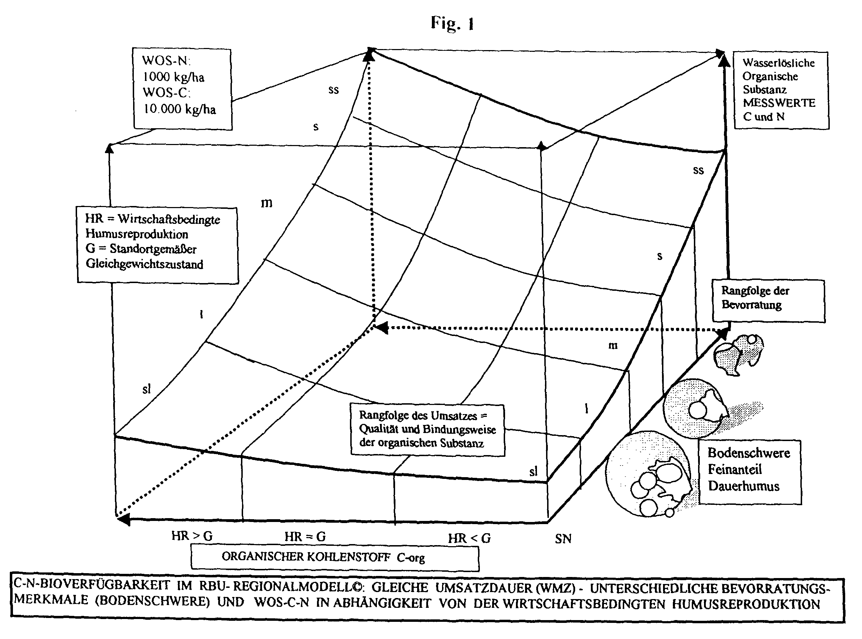

Mit Hilfe der so ermittelten Kalibrationsgleichungen kann bei ausreichender statistischer Absicherung innerhalb ausgewählter Probenkollektive auf die Laboranalyse mit genormten und zeitaufwendigen Verfahren bei einem Großteil der Proben verzichtet werden. Es reicht dann aus, die auf der Kochbank, wie in Abbildung 1 dargestellt, gewonnenen Extrakte mittels geeigneter Geräte zur Nahinfrarotreflexionsspektroskopie bzw. Nahinfrarottransmissionsspektrometrie im Wellenlängenbereich von 800 nm bis 2500 nm zu vermessen und daraus die Gehalte an pflanzenverfügbarem Stickstoff und Boden-Kohlenstoff zu errechnen.

6. Gleichzeitig erfolgt eine Beurteilung sowohl der Gesamtwerte als auch der wasserlöslichen Fraktionen sowie der Rahmenparamter Textur und Wasserkapazität wie folgt, wobei zwischen Angebot und Erwartung in Abhängigkeit vom Standort unterschieden wird:

| Messwert | Ergebnis | Bedeutung für die Nährstoffnachlieferung |
|---|---|---|
| Gesamtwerte (WULOS) Stickstoff Kohlenstoff | nieder | Nur geringes Angebot -geringe Erwartung |
| | hoch | Angebot für hohe Nährstoffversorgung gegeben |
| Fraktion 1 (WOS) | nieder | Wenig Biomasse, geringe Erwartung |
| | hoch | Erwartung hoch |
| Fraktion 2 (WOS) | nieder hoch | Umsetzbarer Gesamtvorrat gering Angebot insgesamt hoch, da hohe Speicherreserve in Biomasse, im Humus und an Ton gebunden |
| Fraktion 3 (WOS) | nieder | Angebot und Erwartung nieder, Bodenlösung verarmt |
| | hoch | Erwartung hoch entsprechend dem Messwert |
| Textur | hoher Feinanteil | hohes Angebot möglich, Erwartung je Klima und Bewirtschaftung |
| Wasserkapazität | hoch | hohe Erwartung gegeben |

Zusätzlich zu der Beurteilung der Werte nach ihrer Höhe erfolgt eine Beurteilung nach ihren Verhältnissen zueinander wie folgt:

| Verhältnisse | Ergebnis | Bedeutung für die Nachlieferung |
|---|---|---|
| C/N der Gesamtgehalte | weit | Erwartung generell niedrig |
| | eng | Erwartung generell hoch |
| C/N der Fraktionen 1-3 | weit | Erwartung niedrig |
| | eng | Erwartung hoch |
| Gesamtgehalte (WULOS) zu Fraktionen (WOS) | weit | Humusqualität gering geringe Erwartung |
| | eng | Humusqualität hoch hohe Erwartung |

Nach der Ermittlung der Gehaltswerte und mitbestimmenden Rahmeneigenschaften wird durch einen Messwertevergleich mit den Erträgen nun eine standortbezogene Einstufung unter Einbeziehung des standörtlichen Klimas mit Hilfe von Entzugsrechnungen und Bilanzierungen der Nährstoffe und Kohlenstoff durchgeführt.

| Zuordnung der Werte in ein Gehaltsklassenschema um den jeweiligen Umsatzprozentsatz der Fraktion 2 zu ermitteln | Kalibrationsmodell (RBU-Modell ©) Berücksichtigung einer Rangfolge in der Bevorratung (Angebot) und im Umsatz (Erwartung) |
|---|---|

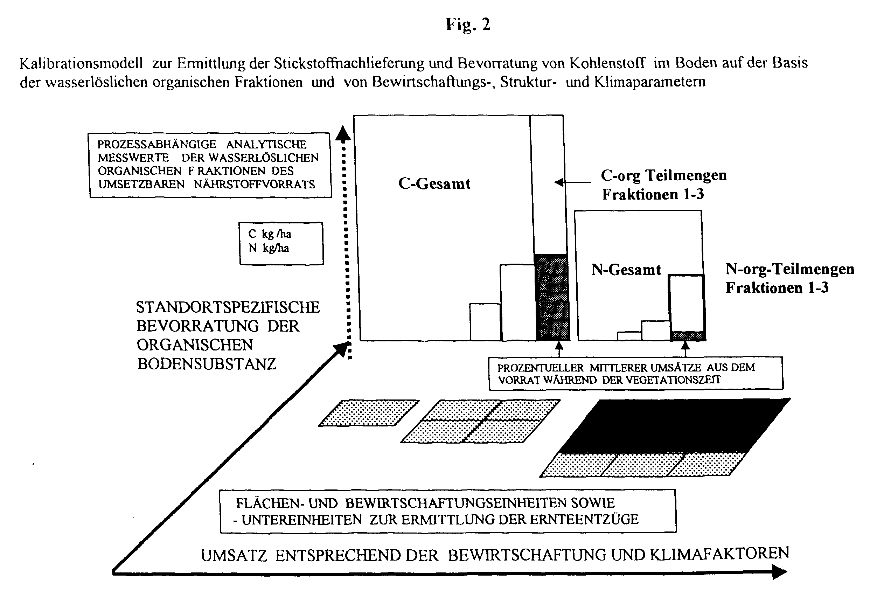

Die einzelnen Messwerte werden je Probe zur Auswertung und Interpretation aufgrund der entsprechenden Standortdaten wie schematisch in Fig. 2 dargestellt, den entsprechenden Flächeneinheiten zugeordnet und in der Folge daraus standortbezogene Gehalts- und Nachlieferungsklassen ermittelt. Die nachstehende Tabelle zeigt ein Einteilungsschema mit jeweils 5 Bevorratungsstufen und 5 Umsatzstufen mit den Rangfolgen sehr nieder, nieder, mittel, hoch, sehr hoch.

**UMSATZSTUFEN : 1-5 steigender Grundumsatz**

| SH : SN | SH : N | SH : M | SH : H | SH : SH |
|---|---|---|---|---|
| H : SN | H : N | H : M | H : H | H : SH |
| M : SN | M : N | M : M | M : H | M : SH |
| N : SN | N : N | N : M | N : H | N : SH |
| SN : SN | SN : N | SN : M | SN : H | SN : SH |

BEVORRATUNGSSTUFEN

Dabei ist davon auszugehen, dass vom jeweiligen Gesamtvorrat an löslicher organischer Substanz (Fraktion 2) ein bestimmter Prozentsatz der Nährstoffkomponente Stickstoff für den Pflanzenbestand zur Verfügung steht.

**Patentansprüche**

1. Verfahren zur Ermittlung der Verfügbarkeit von Stickstoff und/oder Kohlenstoff in Böden und von Erden, Sedimenten und Komposten für das Pflanzenwachstum zur Quantifizierung der Stickstoffdüngung, bei dem aus einer Probe mehrere Teilmengen mittels verschiedener chemischer Verfahren auf den Gehalt an unterschiedlich gebundenen anorganischen Nährstoffen analysiert und die verfügbare Nährstoffmenge berechnet wird, dadurch gekennzeichnet, dass

A) dass das Nachlieferungspotential für pflanzenverfügbaren bzw. organisch gebundenen N und/oder C durch Messung der Gehalte in der wasserlöslichen organischen Substanz (WOS) und wasserunlöslichen organischen Substanz(WULOS) ermittelt,

B) die Bevorratungseigenschaften durch die Humusausstattung mittels Messung des organischen Kohlenstoffgehaltes C gesamt erfasst wird,

C) die Umsatzeigenschaften des Bodens durch Messung der Bodentextur und der Wasserkapazität in Kombination mit den Klimafaktoren ermittelt werden und

D) die nach A)bis C) analysierten Proben zusätzlich mittels Reflexionsspektroskopie und/oder Transmissionsspektroskopie im Wellenlängenbereich von 400 nm bis 2500 nm vermessen werden,

E) dass die Messgrößen in Form der Absorptionswerte bei bestimmten Wellenlängen mittels Regressionsgleichungen kalibriert und quantitativ nach dem Gehalt an Stickstoff und Kohlenstoff durch den rechnerischen Vergleich der Werte aus der/den nasschemisch analysierten Fraktion(en) ausgewertet werden und

F) die ermittelten Beziehungsgrößen in Form von Gruppen- oder Standardkalibrationen für die quantitative Auswertung weiterer Probenmesswerte aus der Reflexionsspektroskopie oder Transmissionsspektroskopie im Wellenlängenbereich von 400 nm bis 2500 nm genutzt werden und abschließend

G) durch Vergleich mit den Erträgen eine standortbezogene Einstufung mittels einer Entzugsrechnung und Bilanzierung der Nährstoffe und Kohlenstoff erfolgt.

**2.** Verfahren nach Anspruch 1, dadurch gekennzeichnet, dass zur Ermittlung des N- und C- gehaltes in der WOS ein Heißwasserextrakt und wahlweise weitere wässerige Extrakte aus der gleichen Probe mittels chemischer Mittel, die über dispergierende Eigenschaften verfügen und gleichzeitig Tauschprozesse begünstigen, parallel analysiert und verglichen werden.

**3.** Verfahren nach Anspruch 1 und 2, dadurch gekennzeichnet, dass zur Ermittlung des N- und C- gehaltes in der WOS anorganische Salzlösungen, vorzugsweise 0,04 mol Phosphatpuffer oder 0,01 bis 0,1 Boratpuffer oder Mischungen aus beiden oder auch Calciumchloridlösungen Verwendung finden.

**4.** Verfahren nach Anspruch 1 bis 3, dadurch gekennzeichnet, dass die Extraktionsrückstände (die Filterkuchen) getrocknet und zur Bestimmung von Gesamt-Reststickstoff und Gesamt-Restkohlenstoff (Wulos) nach standardisierten nasschemischen oder physikochemischen Methoden herangezogen werden.

**5.** Verfahren nach Anspruch 1 bis 4, dadurch gekennzeichnet, dass die Textur und Wasserkapazität mit Hilfe von Standardmethoden ermittelt wird um die Bevorratungs und Umsatzeigenschaften zu definieren.

**6.** Verfahren nach Anspruch 1 dadurch gekennzeichnet, dass die aufbereitete(n) Fraktion(en) mittels Nahinfarotreflexionsspektroskopie und/oder Nahinfarottransmissionsspektroskopie im Wellenlängenbereich von 800 nm bis 2500 nm vermessen werden.

**7.** Verfahren nach Anspruch 1 bis 5, dadurch gekennzeichnet, dass die Fraktionen mit Trägermaterial, vorzugsweise Kieselgel (CELITE ®) vermischt und getrocknet und spektroskopisch vermessen werden.

**8.** Verfahren nach Anspruch 1 und 5 dadurch gekennzeichnet, dass die Bodenproben getrocknet, auf eine einheitliche Korngröße von < 1 mm mit einer Kugelmühle gemahlen und direkt spektroskopisch vermessen werden.

**9.** Verfahren nach Anspruch 1 bis 6 dadurch gekennzeichnet, dass die Proben bzw. Fraktionen nach Stickstoff- und/oder Kohlenstoffgehalt und jenen Parametern spektroskopisch direkt vermessen werden, die deren Umsetzungsvorgänge beeinflussen, wie Textur, Wasserkapazität und dergleichen.

**9.** Verfahren nach Anspruch 1 bis 6, dadurch gekennzeichnet, dass die Extraktionsrückstände (die Filterkuchen) getrocknet und zur Bestimmung von Gesamt-Reststickstoff und Gesamt-Restkohlenstoff sowie von jenen Parametern verwendet werden, die mit dem Nachlieferungsprozess im Zusammenhang stehen, bzw. die die Umsetzungsvorgänge beeinflussen, wie Textur, Wasserkapazität und dergleichen

**10.** Verfahren nach Anspruch 1, 6 und 9, dadurch gekennzeichnet, dass die spektroskopische Messung an Bohrkernen oder direkt vor Ort an der Oberfläche, Pflugsohle usw. erfolgt.

**11.** Verfahren nach Anspruch 1 bis 10, dadurch gekennzeichnet, dass ein Vergleich mit den Pflanzenerträgen bzw. deren Nährstoffentzügen an einem bestimmten Standort und eine Berechnung der Nachlieferung von Stickstoff aus der organischen Substanz mit Hilfe der Größen von analytischen Messwerten und deren Beziehungen zueinander in einem Kalibrationsmodell erfolgt, welches durch Einbeziehung der Bevorratungsleistung und der Umsatzeigenschaften von Böden und dergleichen das Nährstoffangebot und die zu erwartenden Nährstoffmengen qualitativ und quantitativ ermittelt.

Fig. 1

## Fig. 2

Kalibrationsmodell zur Ermittlung der Stickstoffnachlieferung und Bevorratung von Kohlenstoff im Boden auf der Basis der wasserlöslichen organischen Fraktionen und von Bewirtschaftungs-, Struktur- und Klimaparametern

C-org Teilmengen Fraktionen 1-3

N-org-Teilmengen Fraktionen 1-3

C-Gesamt

N-Gesamt

PROZENTUELLER MITTLERER UMSÄTZE AUS DEM VORRAT WÄHREND DER VEGETATIONSZEIT

PROZESSABHÄNGIGE ANALYTISCHE MESSWERTE DER WASSERLÖSLICHEN ORGANISCHEN FRAKTIONEN DES UMSETZBAREN NÄHRSTOFFVORRATS

C kg /ha
N kg/ha

STANDORTSPEZIFISCHE BEVORRATUNG DER ORGANISCHEN BODENSUBSTANZ

FLÄCHEN- UND BEWIRTSCHAFTUNGSEINHEITEN SOWIE - UNTEREINHEITEN ZUR ERMITTLUNG DER ERNTEENTZÜGE

UMSATZ ENTSPRECHEND DER BEWIRTSCHAFTUNG UND KLIMAFAKTOREN

# Fig.3

# Fig. 4

# Fig. 5

**Flußschema Labor-Analytik (Monitoring)-Klassifikation - Spektrencharakteristik**

<u>Bodenmischprobe (3 Tiefenstufen zur Standortdefinition)</u>

\* Wasserlösliche organische Substanz
\*\* Wasserunlösliche organische Substanz
\*\*\* Gesamte organische Substanz